# EUROPÄISCHE PATENTSCHRIFT

(11) **EP 1 109 541 B1**
(45) Veröffentlichungstag und Bekanntmachung des Hinweises auf die Patenterteilung: **19.10.2005**
(21) Anmeldenummer: 99944317.9
(22) Anmeldetag: 31.07.1999
(51) Int. Cl.: A61K 9/70, A61K 9/00, A61K 9/20

(54) **FOLIENFÖRMIGE WIRKSTOFFTRÄGER**
ACTIVE INGREDIENT SUPPORT IN THE FORM OF A FILM
SUPPORT DE PRINCIPE ACTIF, SOUS FORME DE FILM

(30) Priorität: 17.08.1998 DE 19837073
(43) Veröffentlichungstag der Anmeldung: 27.06.2001
(73) Patentinhaber: LTS LOHMANN Therapie-Systeme GmbH, 56567 Neuwied (DE)
(72) Erfinder: ASMUSSEN, Bodo, D-56170 Bendorf-Sayn (DE); HORSTMANN, Michael, D-56564 Neuwied (DE)
(74) Vertreter: Flaccus, Rolf-Dieter
(86) Internationale Anmeldenummer: PCT/EP1999/005549
(87) Internationale Veröffentlichungsnummer: WO 2000/010539

(56) Entgegenhaltungen:
- EP-A- 0 090 560
- EP-A- 0 307 904
- DE-A- 19 800 523
- US-A- 4 128 445
- US-A- 4 812 315

## Beschreibung

Die Erfindung betrifft einen folienförmigen Wirkstoffträger mit auf seiner Oberfläche aufbringbaren Partikeln eines wirkstoffhaltigen Substratsgemäß Anspruch 1.

Im Mundbereich und auf dessen Schleimhäuten anzuwendende Darreichungsformen sind bekannt.

US 3,444,858 beschreibt Medikamentstreifen auf Basis eines gelatineartigen Materials.

Im Dokument "New England Journal of Medicine", Band 289, Seiten 533 bis 535, sind bereits 1937 Arzneimittel in Folienform beschrieben.

Aus DE-OS 24 49 865 sind Arzneimittelwirkstoffträger in Folienform bekannt, die nebeneinander unterschiedliche Wirkstoffe und Wirkstoffkonzentrationen enthalten.

EP 0 219 762 beschreibt eine wasserlösliche Folie aus Stärke, Gelatine, Glycerin und/oder Sorbit, die mittels eines Walzenauftragsverfahren beschichtet wird. Dabei wird erwähnt, daß sich diese Darreichungsformen auch für chemische Reagenzien, Aromastoffe etc. herstellen lassen.

Die kanadische Patentanmeldung 492 040 beschreibt ein verfahren zur Herstellung folienförmiger Zubereitungen unter Einsatz eines Wirkstoffs, zusammen mit Gelatine oder Agar, Gluten, Carboxyvinylpolymer, mehrwertigem Alkohol, einem pflanzlichen Schleim, bzw. Wachs und Wasser.

Die EP 0 460 588 offenbart eine zur Herstellung folienförmiger Systeme geeignete Formulierung. Besonders vorteilhaft ist hierbei eine Zusammensetzung aus 20 bis 60 Gew.% Filmbildner, 2 bis 40 Gew.% Gelbildner, 0,1 bis 35 Gew.% Wirkstoff und maximal 40 Gew.% eines inerten Füllstoffs. Als Gelbildner wird unter anderen Stoffen Polyvinylalkohol genannt.

Nach DE-OS 36 30 603 ist es besonders vorteilhaft, eine flächenhafte Dosierungsform auf einem als Trennfilm ausgebildeten Trägermaterial dosisweise abziehbar aufzubringen.

US 4,128,445 und US 4,197,289 diskutieren mögliche technische Lösungen der Beladung von Trägermaterial mit Wirkstoffen. Beschrieben werden Beladungsverfahren in trockenem und feuchtem Milieu, welche eine gleichmäßige Verteilung von Wirkstoffen auf diesen Schichten zum Ziel haben oder auch auf elektrostatischem Wege erfolgen können.

US Re 31764 offenbart eine detaillierte Darstellung von Möglichkeiten zur tropfenförmigen Applikation pharmazeutischer Wirkstoffe auf flache Substrate. Dabei werden Mechanismen von piezoelektrischem Aufbringen der Wirkstoffe unter Zuhilfenahme elektrischer Felder beschrieben.

US 5,089,307 beschreibt einen heißsiegelbaren, eßbaren Film auf Basis von wasserlöslichen Polysacchariden, mehrwertigen Alkoholen und Wasser. Dabei werden jedoch nur Nahrungsmittelanwendungen, nicht aber Arzneianwendungen beschrieben. Aus US 5,714,007 ist die Beaufschlagung von Oberflächen pharmazeutischer Formulierungen mit pulverförmigen Wirkstoffen bekannt. Dabei wird mittels elektrostatischer Aufladung von Wirkstoffpartikeln bei entgegengesetzter Ladung des Arzneimittelträgers eine über Spannung, Polarität oder Dauer kontrollierte Wirkstoffbeladung ebenflächiger Schichten durchgeführt.

Die US 4,851,394 beschreibt eßbare Filme auf Basis von Glucomannan und Glycerin als Hülle von Weichkapseln oder als beim Verzehr auflösbare Lebensmittelverpackungen.

Die US 5,232,704 offenbart orale Arzneiformen, bei welchen eine Auftriebskraft durch mittels Flüssigkeitszutritt erfolgende Gaserzeugung als Mittel einer verlängerten Verweilzeit im Magen mit dem Ziel einer verlängerten Wirkungsdauer eingesetzt wird.

Die US 5,047,244 beschreibt folienförmige Systeme mit vorteilhaft zweischichtigem Aufbau aus einer wasserquellbaren Schicht und einem wasserunlöslichen Barrierefilm. Das Dokument berichtet über die Verwendung von Polymeren wie Polyethylenglycol, Einsatz von kolloidalem Siliciumdioxid, bioadhäsiven, z.B. carboxyfunktionellen Polymeren, Polyvinylalkohol und anderen Hilfsstoffen.

Die vorstehend beschriebenen Zubereitungen erfüllen jedoch nicht oder nur höchst unvollkommen die nachstehenden Forderungen an Arzneiformen der eingangs genannten Art. Diese Forderungen beinhalten:
- die Wirkstoffdosierung muß äußerst exakt und genau reproduzierbar zubereitbar sein;
- die fertige Arzneiform muß den Wirkstoff unveränderbar in Menge und Qualität enthalten und soll in gewünschter mechanischer Stabilität vorliegen;
- die Arzneiformen müssen den Wirkstoff in vorbestimmbarer Weise nicht unmittelbar und unkontrollierbar, sondern gleichmäßig über einen längeren Zeitraum an den Organismus abgeben;
- bei oraler Applikation muß die Verweildauer im Magen nach Maßgabe einer erwünschten Wirkungsdauer erhöht sein;
- die Auflösungsgeschwindigkeit schwerlöslicher Wirkstoff-Bestandteile soll erhöht sein;
- bei Verwendung von Folienstücken als fertige Arzneiform zur oralen Applikation sollen diese ihre flächenhafte Form behalten und eine vielfach bei gegossenen Folien zu erwartende Aufrollung vermeiden,

Diese Forderungen werden von den zum Stand der Technik genannten Systemen und Formulierungen nicht zufriedenstellend gelöst. Infolgedessen liegt der Erfindung die Aufgabe zugrunde, einen folienförmigen Wirkstoffträger der im Oberbegriff von Anspruch 1 genannten Art anzugeben, welcher sowohl eine exakte und genau reproduzierbare Wirkstoffdosierung als auch eine exakte und genau reproduzierbare Wirkstoffabgabe in einer vorgesehenen Abgaberate ermöglicht und den Wirkstoff gegen vorzeitige Ablösung geschützt auf bzw. in einem mechanisch stabilen folienförmigen Wirkstoffträger enthält.

Die Aufgabe wird durch einen folienförmigen Wirkstoffträger gemäß Anspruch 1 gelöst.

Die Aufgabe wird bei einem folienförmigen Wirkstoffträger der eingangs genannten Art mit der Erfindung dadurch gelöst, daß die Partikel des wirkstoffhaltigen Substrats mit diesem eine feste Verbindung aufweisen.

Dabei sieht eine Ausgestaltung der Erfindung vor, daß die Partikel mit Hilfe eines Lösemittels auf diesem fixiert sind.

Dabei erfolgt vorteilhaft die Verankerung der Partikel auf der Trägerfolie durch vorübergehende Wechselwirkung mit Feuchtigkeit oder Lösemitteldampf oder unter Einwirkung von Druck und/oder Temperatur.

Es wird von der Maßnahme Gebrauch gemacht, daß durch Aufbringen einer weiteren folienförmigen Schicht eine Einkapselung der Arzneistoffpartikel erreicht wird.

Die Aufbringung dieser zusätzlichen Schicht kann durch Kalandrieren mit oder ohne Anwendung von Lösemittel oder Wärme erfolgen. Vorteilhaft kann auch durch die Verwendung schmelzbarer Partikel einer wirkstoffhaltigen Zubereitung deren Haftung auf bzw. in der Trägerschicht optimal verwirklicht werden. Es ist von erfindungswesentlicher Bedeutung, daß die weitere folienförmige Schicht wasserdampfdiffusibel ist. Dabei werden durch die aufgebrachte zweite folienförmige Schicht die oberflächlich auf einen Träger aufgebrachten Partikel zusätzlich geschützt und bei der Wahl einer geeigneten Folie die Abgaberate des Wirkstoffs gesteuert, indem sich dabei vorherbestimmbare und insbesondere verzögerte Wirkstoffabgabecharakteristika einstellen lassen.

Wenn dabei die beiden zur Laminierung verwendeten Folien in der Randkontur aufeinander versiegelt werden, kann durch Einsatz gasbildender Komponenten z.B. in wäßrigem Milieu das Laminat als gasgefüllter schwimmfähiger Beutel im Magen als gastroretentives System eingesetzt werden. Eine weitere Verzögerung der Wirkstoffabgabe wird dadurch erreicht daß in dem durch Gasentwicklung aufgeblähten Raum des Wirkstoffträgers wenigstens ein weiteres folienförmiges Gebilde enthalten ist, auf dessen Oberfläche wirkstoffhaltige Partikel vorhanden sind, die mit dem Gebilde eine feste Verbindung aufweisen.

Weiterhin können im Hohlraum des Wirkstoffträgers zusätzliche folienförmige Gebilde vorhanden sein, die als Hilfsstoffträger zur Gaserzeugung ausgebildet sind.

Die Verwendung des folienförmigen Wirkstoff trägers ist als Darreichungsform für kosmetische, pharmazeutische oder lebensmitteltechnische Produkte vorgesehen.

Weitere Einzelheiten und Vorteile der Erfindung ergeben sich aus der nachstehenden Erläuterung einiger in den Zeichnungen schematisch dargestellter Ausführungsbeispiele. Es zeigen:
Figur 1 bis Figur 6 im Schnitt und in der Seitenansicht unterschiedliche, jedoch ähnliche Ausführungen folienförmiger Wirkstoffträger.
Figur 1 zeigt im Schnitt einen nicht-erfindungsgemäßen folienförmigen Wirkstoffträger (2), bestückt mit auf diesem fest verankerten Partikeln (1) eines wirkstoffhaltigen Substrats. Dabei kann von der Maßnahme Gebrauch gemacht sein, daß bei schwerlöslichen Arzneistoffen eine mikronisierte Form des Wirkstoffs bei Korngrößen von unter 50 µm, bevorzugt unter 5 µm, homogen auf der Oberfläche aufgebracht und unter Vermeidung von Agglomeration auf der Oberfläche fixiert wird. Dabei kann für den folienförmigen Arzneiträger eine leicht wasserlösliche Grundlage gewählt werden.

Figur 2 zeigt eine nicht-erfindungsgemäße Ausgestaltung des folienförmigen Wirkstoffträgers mit Wirkstoffteilchen (1), dem folienförmigen Träger (2) und einem weiteren aufkaschierten folienförmigen Träger (3).

Figur 3 zeigt eine nicht-erfindungsgemäße Ausgestaltung des folienförmigen Wirkstoffträgers (2) mit mit dessen Oberfläche fest verbundenen Wirkstoffteilchen (1), der unter Ausbildung eines Hohlraumes (4) mit einer folienförmigen Schicht (3) überdeckt bzw. eingekapselt ist.

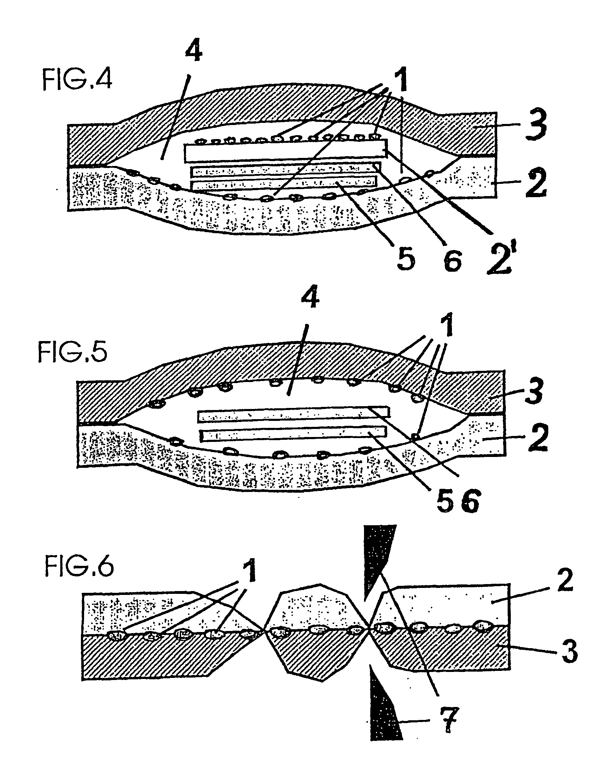

Nach der Figur 4 bildet der folienförmige Wirkstoffträger (2) mit der ihn überdeckenden folienförmigen Schicht (3) einen Hohlraum (4), in welchem ein zusätzliches folienförmiges Element (2') mit auf dessen Oberfläche fixierten Wirkstoffpartikeln (1) eingelagert ist. Im Hohlraum (4) sind folienförmige Hilfsstoffträger (5, 6) zur Gaserzeugung vorgesehen. Für deren Aktivierung durch Zutritt von Feuchtigkeit ist die folienförmige Schicht (3) mit wasserdampfdiffusiblem Material ausgebildet. Der Wirkstoffträger (2) kann mit auf seiner Oberfläche fixierten Wirkstoffpartikeln bestückt sein.

Wie die Figur 5 zeigt, ist eine weitere alternative Ausgestaltung der Vorrichtung nach der Erfindung mit einem folienförmigen Wirkstoffträger (2) und einer wasserdampfdiffusiblen Schicht (3) unter Ausbildung eines Hohlraumes (4) ausgebildet, in dem sich folienförmige Hilfsstoffträger (5, 6) befinden. Die Wirkstoffpartikel (1) sind auf den inneren Flächen der Schichten (2, 3) fixiert.

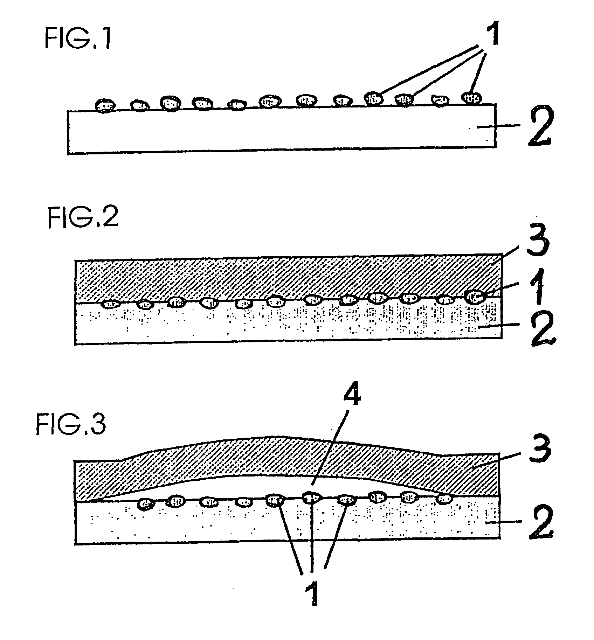

Figur 6 zeigt die Vereinzelung eines aus den Schichten (2, 3) bestehenden, mit Wirkstoffpartikeln (1) bestückten Stranges unter Verwendung einer Trennvorrichtung (7).
Die in den Figuren 1 bis 6 gezeigten Arzneimittelträger (2, 2') bestehen aus Folie filmbildender Materialien wie beispielsweie Polyvinylalkohol, bevorzugt in teilhydrolysierter Form, bei der zwischen 1 und 20 %, besonders bevorzugt 12 % der Hydroxylgruppen durch Acetylgruppen ersetzt sind, Cellulosederivaten, Gelatine, Pullulan oder anderen Sacchariden, pflanzlichen Gummen und Polyvinylpyrrolidon. Weiter können zur Regulation der Auflöseeigenschaften mit Vorteil Zucker, Glucose, Sorbit, Polyethylenglycol und andere wasserlösliche Zusatzstoffe zugesetzt werden. Zusätze von bis zu 30 Gew.% einer oberflächenaktiven Substanz können die Benetzung zu den wirkstoffhaltigen Partikeln (1) verbessern.
Eine Zugabe von bis zu 40 Gew.% eines Füllstoffs hebt die erfindungsgemäßen Vorteile nicht auf und eignet sich ebenfalls zur Moderation der Auflösungseigenschaften. Hierzu eignen sich ohne Anspruch auf erschöpfende Aufzählung Siliciumdioxid, Titandioxid, Calciumcarbonat, Calciumsulphat, Calciumphosphat, Talkum oder Mischungen dieser Stoffe. Zur Verbesserung der Akzeptanz können auch Aromen zugesetzt werden, die vorteilhaft eine tropfenförmige Innenphase in der Schicht bilden.
Weiterhin sind aromaunterstützende Stoffe wie Natriumsaccharinat, andere Süßstoffe, Salz oder Zuckerderivate zur Verbesserung des geschmacklichen Empfindens einsetzbar, wie z.B. auch niedermolekulare organische Säuren, beispielsweise Apfelsäure, Adipinsäure oder Zitronensäure. Weiterhin verbessern Stabilisatoren wie Antioxidantien die Lagerfähigkeit der anhaftenden Wirkstoffpartikel.

Diese können aus den weiter oben genannten Grundbestandteilen der Folie selbst, aber auch aus mehr lipophilen, z.B. wachs- oder harzartigen Anteilen bestehen.
Thermoplastische Komponenten, auch wasserlösliche Polymere wie Polyethylen, eignen sich zur Einbettung nach Aufbringung auf eine Trägerfolie.
In der Regel dürfen die Wirkstoffe selbst in reiner und gegebenenfalls mikronisierter Form auf einen Träger aufgebracht werden.
Die folienförmigen Produkte oder Vorprodukte weisen bevorzugt eine Dicke von 20 bis 300 µm auf; ihre Größe beträgt vorteilhaft 0,5 bis 8 cm².

Durch das erfindungsgemäße Aufbringen einer zweiten folienförmigen Schicht (3) auf einen Wirkstoffträger (2) können die oberflächig aufgebrachten Partikel (1) geschützt und eingekapselt werden. Dies kann - je nach Grundmaterial sowie Temperaturempfindlichkeit - durch Heißkalandrieren, Kleben unter Zuhilfenahme von Lösemitteln oder andere, dem Fachmann bekannte Techniken erfolgen.

Durch Weiterverarbeitung z.B. mittels Konturwalzen oder ähnliche formgebende Vorrichtungen kann das Folienlaminat eine gewünschte geometrische Kontur erhalten, beispielsweise eine zur Füllung in Kapseln geeignete Form. Damit lassen sich z.B. deutlich verzögerte Wirkstoff-Abgabecharakteristika erzielen.
Wenn zwei zur Laminierung vorgesehene Folien in der Randkontur miteinander versiegelt werden, können damit weitere besondere Wirkungen erreicht werden.

Einerseits kann damit eine extreme Verzögerung der Wirkstoffabgaberate verwirklicht werden, und andererseits kann das Laminat durch Einsatz gasbildender Komponenten im Innenraum einer als Beutel ausgebildeten erfindungsgemäßen Vorrichtung mit wasserdampfdiffusiblen Schichten als gastroretentives Therapeutikum im Magen- bzw. Intestinaltrakt eingesetzt werden.

Der folienförmige Wirkstoffträger nach der Erfindung ist vielseitig verwendbar, in industriellen Produktionsprozessen mit hohem Ausbringen bei moderaten Kosten herstellbar und löst in optimaler Weise die eingangs gestellte Aufgabe.

## Patentansprüche

1. Folienförmiger Wirkstoffträger zur gesteuerten Wirkstoffabgabe, umfassend eine Trägerfolie, mit deren Oberfläche Partikel eines wirkstoffhaltigen Substrats in fester Verbindung stehen oder in die die Partikel eingebettet sind, sowie eine weitere folienförmige Schicht, welche die mit Wirkstoffpartikeln angereicherte Trägerfolie überdeckt, wobei die weitere folienförmige Schicht wasserdampfdiffusibel ist und die Trägerfolie und die weitere folienförmige Schicht in ihrer Randkontur aufeinander versiegelt sind, so daß der folienförmige Wirkstoffträger als Beutel ausgebildet ist, in dessen Innenraum gasbildende Komponenten vorhanden sind, **dadurch gekennzeichnet, daß** im Hohlraum des Wirkstoffträgers wenigstens ein weiteres folienförmiges Gebilde enthalten ist, auf dessen Oberfläche wirkstoffhaltige Partikel vorhanden sind, die mit dem Gebilde eine feste Verbindung aufweisen, und/oder im Hohlraum zusätzliche folienförmige Gebilde vorhanden sind, die als Hilfsstoffträger zur Gaserzeugung ausgebildet sind.

2. Folienförmiger Wirkstoffträger nach Anspruch 1, **dadurch gekennzeichnet, daß** als Wirkstoffpartikel Partikel eines schwerlöslichen Wirkstoffs in einer Korngröße von unter 50 µm, vorzugsweise unter 5 µm, aufgebracht sind.

3. Verfahren zur Herstellung eines folienförmigen Wirkstoffträgers zur gesteuerten Wirkstoffabgabe, umfassend eine Trägerfolie, mit deren Oberfläche Partikel eines wirkstoffhaltigen Substrates in fester Verbindung stehen oder in die die Partikel eingebettet sind, sowie eine weitere folienförmige Schicht, die wasserdampfdiffusibel ist und die die mit Wirkstoffpartikeln angereicherte Trägerfolie überdeckt, wobei diese Partikel mit der Oberfläche des folienförmigen Wirkstoffträgers fest verbunden oder in diese eingebettet und unter Einwirkung von Druck und/oder Wärme auf der Trägerfolie verankert werden, wobei die Trägerfolie und die weitere folienförmige Schicht in ihrer Randkontur aufeinander versiegelt werden, so daß der folienförmige Wirkstoffträger als Beutel ausgebildet ist und gasbildende Komponenten in den Innenraum des als Beutel ausgebildeten Wirkstoffträgers eingesetzt werden, **dadurch gekennzeichnet, daß** wenigstens ein weiteres folienförmiges Gebilde, auf dessen Oberfläche wirkstoffhaltige Partikel vorhanden sind, die mit dem Gebilde eine feste Verbindung aufweisen, und/oder zusätzliche folienförmige Gebilde, die als Hilfsstoffträger zur Gaserzeugung ausgebildet sind, in den Hohlraum des als Beutel ausgebildeten Wirkstoffträgers eingesetzt werden.

4. Verfahren nach Anspruch 3, **dadurch gekennzeichnet, daß** als Wirkstoffpartikel Partikel eines schwerlöslichen Wirkstoffs in einer Korngröße von unter 50 µm, vorzugsweise von unter 5 µm aufgebracht werden.

5. Verwendung eines folienförmigen Wirkstoffträgers nach Anspruch 1 oder 2, oder eines Wirkstoffträgers, der durch ein Verfahren nach einem der Ansprüche 3 oder 4 hergestellt wurde, als Darreichungsform für kosmetische, pharmazeutische oder lebensmitteltechnische Produkte.

## Claims

1. Film-shaped active substance carrier for controlled release of active substance, comprising a carrier film to the surface of which particles of an active substance-containing substrate are fixedly connected or into the surface of which the said particles are embedded, as well as a further film-shaped layer covering the said carrier film, which carrier film is enriched with active substance particles, the said further film-shaped layer being water vapor-diffusible, and the said carrier film and the said further film-shaped layer being sealed on top of one another in their edge contour such that the film-shaped active substance carrier is configured as a pouch in whose interior space there are gas-forming components, **characterized in that** in the cavity of the active substance carrier there is contained at least one further film-shaped body on whose surface there are located active substance-containing particles which are fixedly connected with the said film-shaped body, and/or that in the said cavity there are additional film-shaped bodies which are configured as active substance carriers for the generation of gas.

2. Film-shaped active substance carrier according to Claim 1, **characterized in that** as active substance particles there are applied particles of a slightly soluble active substance which have a particle size of below 50 µm, preferably below 5 µm.

3. Process for manufacturing a film-shaped active substance carrier for the controlled release of active substance, comprising a carrier film, to the surface of which particles of an active substance-containing substrate are fixedly connected or into the surface of which said particles are embedded, and a further film-shaped layer which is water vapor-diffusible and covers the carrier film, said carrier film being enriched with active substance particles, wherein said particles are fixedly connected to the surface of the film-shaped active substance carrier or are embedded therein, and anchored on the carrier film under the action of pressure and/or heat, and wherein said carrier film and the said further film-shaped layer are sealed on top of one another in their edge contour such that the film-shaped active substance carrier is configured in the form of a pouch, and gas-forming components are introduced into the interior space of the active substance carrier configured as a pouch, **characterized in that** at least one further film-shaped body on whose surface there are located active substance-containing particles which are fixedly connected with the said body, and/or additional film-shaped bodies which are configured as active substance carriers for the generation of gas are introduced into the cavity of the active substance carrier, which is configured as a pouch.

4. Process according to claim 3, **characterized in that** as active substance particles there are applied particles of a slightly soluble active substance which have a particle size of below 50 µm, preferably below 5 µm.

5. Use of a film-shaped active substance carrier according to claim 1 or 2, or of an active substance carrier manufactured by means of a process according to claim 3 or 4, as an administration form for cosmetic, pharmaceutical or food-technological products.

## Revendications

1. Support de principe actif sous forme de feuille pour la libération contrôlée de principe actif, comprenant une feuille support, à la surface de laquelle des particules d'un substrat contenant le principe actif se trouve liée solidement ou dans laquelle les particules sont enfouies, ainsi qu'une couche supplémentaire en forme de feuille, laquelle recouvre la feuille support enrichie en particules de principe actif, la couche supplémentaire en forme de feuille étant diffusible par la vapeur d'eau, et la feuille support et la couche supplémentaire en forme de feuille étant scellées l'une sur l'autre, dans leur contour de bord, de sorte que le support de principe actif en forme de feuille est formé en tant que sachet, à l'intérieur duquel sont présents des composants formant des gaz, **caractérisé en ce que**, dans l'espace creux du support de principe actif, est au moins contenue une structure supplémentaire en forme de feuille, sur la surface de laquelle sont présentes des particules contenant le principe actif, qui présentent une liaison solide à la structure, et/ou dans l'espace creux sont présentes des structures supplémentaires en forme de feuille, qui sont formées en tant que support d'adjuvant pour produire des gaz.

2. Support de principe actif en forme de feuille selon la revendication 1, **caractérisé en ce que** des particules d'un principe actif peu soluble, en tant que particules de principe actif, sont appliquées en une granulométrie inférieure à 50 µm, de préférence inférieure à 5 µm.

3. Procédé de préparation d'un support de principe actif en forme de feuille pour la libération contrôlée de principe actif, comprenant une feuille support, à la surface de laquelle sont liées solidement des particules d'un substrat contenant un principe actif, ou dans laquelle les particules sont enfouies, ainsi qu'une couche supplémentaire en forme de feuille, qui est diffusible par la vapeur d'eau, et qui recouvre la feuille support enrichie en particules de principe actif, ces particules étant liées solidement à la surface du support de principe actif en forme de feuille, ou étant enfouies dans cette dernière, et étant ancrées sur la feuille support sous l'action de la pression et/ou de la chaleur, la feuille support et la couche supplémentaire en forme de feuille étant scellées l'une sur l'autre dans leur contour de bord, de sorte que le support de principe actif en forme de feuille est formé en tant que sachet, et des composants formant des gaz sont mis en oeuvre à l'intérieur du support de principe actif formé en tant que sachet, **caractérisé en ce que**, au moins une structure supplémentaire en forme de feuille sur la surface de laquelle sont présentes des particules contenant le principe actif, qui présentent une liaison solide à la structure, et/ou des structures supplémentaires en forme de feuille, qui sont formées en tant que support d'adjuvant pour produire des gaz, sont mises en oeuvre dans l'espace creux du support de principe actif formé en tant que sachet.

4. Procédé selon la revendication 3, **caractérisé en ce que** des particules d'un principe actif peu soluble, en tant que particules de principe actif, sont appliquées en une granulométrie inférieure à 50 µm, de préférence inférieure à 5 µm.

5. Utilisation d'un support de principe actif en forme de feuille selon la revendication 1 ou 2, ou d'un support de principe actif, qui a été fabriqué à l'aide d'un procédé selon l'une quelconque des revendications 3 ou 4, en tant que forme d'administration pour des produits cosmétiques, pharmaceutiques ou produits de l'industrie des denrées alimentaires.
